# EUROPÄISCHE PATENTSCHRIFT

(11) **EP 1 386 591 B1**
(45) Veröffentlichungstag und Bekanntmachung des Hinweises auf die Patenterteilung: **23.03.2005**
(21) Anmeldenummer: 02016564.3
(22) Anmeldetag: 24.07.2002
(51) Int. Cl.: A61F 2/30

(54) **Verfahren zur Herstellung eines Implantats und Verfahren zum Dekontaminieren einer mit Strahlpartikeln behandelten Oberfläche**
Method for manufacturing an implant and method for decontamination of a particle-blasted surface
Méthode pour la fabrication d'un implant et méthode pour la décontamination d'une surface par un jet de particules

(43) Veröffentlichungstag der Anmeldung: 04.02.2004
(73) Patentinhaber: Zimmer GmbH, 8404 Winterthur (CH)
(72) Erfinder: Windler, Markus, 8354 Hofstetten (CH)
(74) Vertreter: Manitz, Finsterwald & Partner GbR

(56) Entgegenhaltungen:
- US-A- 5 057 108
- US-A- 5 344 494
- US-A- 5 826 586
- US-A- 6 025 536
- US-A- 6 095 817

## Beschreibung

Die Erfindung betrifft ein Verfahren zur Herstellung eines Implantats und ein Verfahren zum Dekontaminieren einer mit Strahlpartikeln behandelten Oberfläche.

Bei Knochenimplantaten, wie z.B. Gelenkimplantaten, werden die Oberflächen, die im Knochenmaterial verankert werden, mit Strahlpartikeln behandelt, um die Oberflächen aufzurauhen. Bei dem Strahlen werden einem durch eine Düse geführten Gasstrom feste Partikel in Form von Körnern beigemischt und unter vorgegebenen Winkeln auf die Oberfläche geschossen. Dadurch wird eine rauhe Oberflächenstruktur erzeugt, so dass beispielsweise bei Schaftprothesen, die direkt mit dem Schaft im Knochen verankert werden, das Knochenmaterial bis an die Hinterschneidungen der Oberfläche formschlüssig heranwachsen kann. Auch bei einzementierten Implantaten, wie Schaftprothesen oder Gelenkschalen, ist eine rauhe Oberflächenstruktur erwünscht, um dem Knochenzement einen besseren Halt zu geben. Zudem werden die Prothesenoberflächen durch die Bombardierung mit den Partikeln verfestigt und sind unter der so erzeugten Eigenspannung bei Wechselbelastungen viel weniger rissempfindlich. Dies ist insbesondere bei einer Fermurschaftprothese mit einem schlanken Hals wichtig. Auch an nicht zu verankernden Stellen kann ein Implantat aufgerauht werden, um dem Arzt beim Einsetzen einer Prothese eine zusätzliche Griffigkeit zu vermitteln.

Bekannt ist das Strahlen von Implantatoberflächen mit Korund-Partikeln. Derartig gestrahlte Oberflächen weisen jedoch auch nach der Behandlung in Reinigungsbädern Partikel auf, die in die Oberfläche eingedrungen und dort steckengeblieben sind bzw. an der Oberfläche haften. Derartige Partikel können sich nach der Implantation nachteilig auswirken, beispielsweise wenn sie sich mechanisch lösen, zwischen die Artikulationsflächen eines künstlichen Gelenks gelangen, dessen Standzeit herabsetzen und daher zu den gleichen negativen Wirkungen wie beispielsweise ein Knochenzementabrieb führen können. Aus US 6,095,817 ist es bekannt, zum Aufrauen eingesetztes Strahlmaterial durch Behandeln mit einem Lösungsmittel zu entfernen, wenn das betreffende Implantat beschichtet werden soll und das Lösen des zuvor verwendeten Strahlmaterials nur dazu dient, die Anhaftung der Beschichtung nicht zu gefährden, wobei Strahl- und Beschichtungsmaterial identisch sind.

Aufgabe der Erfindung ist es, ein Verfahren zur Herstellung eines Implantats anzugeben, dessen Oberfläche mit Strahlen aufgerauht ist, jedoch nicht mit dem verwendeten Strahlmaterial kontaminiert ist.

Die Lösung der Aufgabe erfolgt durch die Merkmale des Anspruchs 1 sowie des Anspruchs 2.

Vorteilhafte Ausführungsformen der Erfindung sind in der Beschreibung und in den Unteransprüchen angegeben.

Erfindungsgemäß wird ein Implantat mit einer Oberfläche hergestellt, die durch das Strahlen die gewünschte rauhe Oberflächenstruktur erhält, jedoch nicht mit den Strahlpartikeln, die beim Strahlen in die Oberfläche eingebettet werden oder an ihr haften und sich nach der Implantation der Prothese lösen können, verunreinigt ist. Das selektive Lösen der Strahlpartikel führt zudem zu dem Vorteil, dass die aufgerauhte Oberfläche durch das Behandeln mit dem Lösungsmittel nicht wesentlich verändert wird.

Erfindungsgemäß kann jegliche mit Strahlpartikeln behandelte Oberfläche, insbesondere eine Implantatoberfläche, von den Strahlpartikeln, die nach dem Strahlen in und an der Oberfläche verbleiben, befreit und somit dekontaminiert werden.

Die Erfindung besteht folglich in der Kombination aus einer Implantatoberfläche, Strahlpartikeln und wenigstens einem Lösungsmittel, wobei diese Bestandteile hinsichtlich ihrer relevanten Eigenschaften derart aufeinander abgestimmt sind, dass die Strahlpartikel die Oberfläche in der gewünschten Weise aufrauhen können und das Lösungsmittel einerseits die Strahlpartikel lösen kann, gleichzeitig aber die Oberfläche nicht weiter angreift.

Das Behandeln der aufgerauhten Oberfläche mit Lösungsmitteln wird auch als Beizen bezeichnet.

Als Lösungsmittel kann ein säurehaltiges Lösungsmittel verwendet werden, in dem das Material des Implantats im wesentlichen nicht löslich ist. Die Säure ist dabei ein aktives Ingredienz, das die Strahlpartikel von der Oberfläche löst bzw. diese auflöst. So kann durch eine einfache Behandlung mit einem säurehaltigen Lösungsmittel die Oberfläche dekontaminiert werden, ohne dass umständliche Reinigungsprozesse erforderlich sind.

Erfindungsgemäß ist vorgesehen, dass das Material der Oberfläche Titan oder eine Titan-Legierung aufweist, dass die Strahlpartikel Eisenpartikel umfassen, und dass das Lösungsmittel HNO₃ aufweist. Dadurch können in einem einfachen, problemlos beherrschbaren Prozess die metallischen Strahlpartikel von der Oberfläche abgelöst bzw. gelöst werden.

Das Behandeln der Oberfläche kann ein Eintauchen der Oberfläche in die mechanisch gerührte Säure umfassen. Dies gewährleistet einen Lösungsvorgang, bei dem alle Bereiche der Oberfläche mit dem Lösungsmittel behandelt und dekontaminiert werden können.

Vor dem Behandeln mit dem Lösungsmittel kann eine Reinigung der Oberfläche erfolgen. Dadurch können Verunreinigungen der Oberfläche, die eine Benetzung oder Oberfläche mit dem Lösungsmittel verhindern, entfernt werden, so dass ein vollständiges Auflösen bzw. Ablösen der Strahlpartikel erfolgen kann.

Das Lösungsmittel kann beim Behandeln der Oberfläche aufbereitet werden. So bleibt das Lösungsmittel auch bei längeren Behandlungsprozeduren aktiv und die Lösungsreaktion wird günstig beeinflusst. Die Aufbereitung des Lösungsmittels kann insbesondere durch Ausfällen der gelösten Strahlpartikel erfolgen.

Es können durch das erfindungsgemäße Verfahren Oberflächen behandelt und dekontaminiert werden, die sowohl aus einem Metall als auch aus einer Zusammensetzung einer Mehrzahl metallischer Elemente bestehen und durch übliche Verfahren nur schwierig von den Strahlpartikeln befreit werden können.

Die Strahlpartikel umfassen Eisenpartikel, die insbesondere gehärtet sein können. Diese, mit bekannten Verfahren nur schwierig entfernbaren Partikel können durch das erfindungsgemäße Verfahren selektiv gelöst werden.

In einem besonders bevorzugten Ausführungsbeispiel kann die Oberfläche Titan oder eine Titanlegierung umfassen, die Strahlpartikel können Eisenpartikel umfassen, und das Behandeln der Oberfläche kann ein bis dreistündiges Eintauchen der Oberfläche in eine mechanisch gerührte, etwa 10 %-ige HNO₃ als Lösungsmittel bei Raumtemperatur umfassen. Diese Kombination der Strahlpartikel und des Lösungsmittels gewährleistet, dass selbst eine Oberfläche, die aus Titan oder einer Titanlegierung besteht, eine ausreichende Oberflächenrauhigkeit erhält und außerdem von den zum Aufrauhen benötigten Eisenpartikeln befreit wird, und zwar derart, dass das Grundmaterial nicht in nachteiliger Weise angegriffen wird.

Dabei kann vor dem Behandeln der Oberfläche eine Reinigung der Oberfläche mit Industriealkohol erfolgen und/oder beim Behandeln der Oberfläche das Lösungsmittel durch eine Fällung der gelösten Strahlpartikel, insbesondere durch eine Eisenfällung, aufbereitet werden. So kann bei der Behandlung einer Oberfläche aus Titan oder einer Titanlegierung, die mit Eisenpartikeln aufgerauht wird, durch die Reinigung der Oberfläche mit Industriealkohol vor dem Behandeln der Oberfläche mit einem Lösungsmittel eine ausreichende Benetzbarkeit der Oberfläche mit dem Lösungsmittel bewirkt werden. Zudem wird durch die Eisenfällung während der Behandlung der Oberfläche das Lösungsmittel bzw. die Salpetersäure regeneriert, wodurch das Auflösen der Eisenpartikel günstig beeinflusst wird.

Ein erfindungsgemäß hergestelltes medizinisches Implantat ist durch Strahlen mit Strahlpartikeln aufgerauht, so dass bei verankerten Schaftprothesen Knochenmaterial bis an die Hinterschneidungen der Oberfläche formschlüssig heranwachsen kann, bei einzementierten Implantaten dem Knochenzement ein besserer Halt gegeben werden kann und zugleich die Oberfläche durch die Bombardierung mit den Partikeln verfestigt wird. Gleichzeitig ist die aufgerauhte Oberfläche selektiv von den unerwünschten Strahlpartikeln befreit, so dass eine mechanische Ablösung von in und auf der Oberfläche verbliebenen Strahlpartikeln nach der Implantation vermieden werden kann.

Weitere Vorteile der Erfindung liegen darin, dass sie einfach anwendbar und kostengünstig ist, um mit Sicherheit eine nahezu strahlmittelfreie, durch Strahlen aufgerauhte Oberfläche zu erhalten und Komplikationen, die im implantierten Zustand durch "vagabundierende" Strahlpartikel entstehen können, vorzubeugen. Ferner ist von Vorteil, dass das Beizen der Oberfläche bei Raumtemperatur und in vergleichsweise kurzer Zeit (bei den bevorzugten Ausführungsbeispielen in maximal 3 Stunden) erfolgen kann.

Nachfolgend wird die Erfindung rein beispielhaft beschrieben.

In einer Ausführungsform des erfindungsgemäßen Verfahrens wird die Oberfläche eines Implantats, die aus Titan oder einer Titanlegierung besteht, durch Strahlen mit gehärteten Eisenpartikeln, die aus kantigen, gebrochenen Stahlgussstrahlkörnern großer Härte bestehen, beschossen. Die Korngröße der gehärteten Eisenpartikel beträgt etwa 0,07 bis 1,7 mm, vorzugsweise 0,1 bis 0,4 mm zum Erreichen einer Rauhigkeit Ra, die zwischen 0,5 und 14 µm liegen kann. Dabei wird ein Strahldruck von etwa 1 bis 6 bar und ein Abstand der zum Strahlen benutzten Düse zur Implantatoberfläche von etwa 200 mm eingehalten. Die Proben werden solange bestrahlt, bis die Oberfläche den gewünschten Rauhigkeitsgrad erhält, wobei sie gleichmäßig mattgrau erscheint. Anschließend wird die Oberfläche des Implantats in eine mechanisch gerührte Lösung einer etwa 10 %-igen Salpetersäure (HNO₃) bei Raumtemperatur für ungefähr 30 Minuten eingetaucht. Dabei werden die nach dem Strahlvorgang in oder an der Oberfläche verbliebenen Eisenpartikel aufgelöst bzw. abgelöst, ohne dass die Oberfläche aus Titan oder aus der Titanlegierung angegriffen wird.

Somit wird durch die Wahl einer geeigneten Dreierkombination aus Oberflächenmaterial, Strahlpartikel-Material und Lösungsmittel die Möglichkeit geschaffen, nach dem Aufrauhen der Implantatoberfläche durch Strahlen die Strahlpartikel selektiv mit dem Lösungsmittel von der Oberfläche zu entfernen, so dass eine Implantatoberfläche erhalten wird, die im wesentlichen frei von Strahlpartikeln ist und auf diese Weise dekontaminiert ist.

Bei dem Verfahren dieser Ausführungsform kann vor dem Behandeln der Oberfläche mit der Salpetersäure eine Reinigung der aufgerauhten Oberfläche mit Industriealkohol durchgeführt werden, so dass diese im nachfolgenden Schritt von der Salpetersäure vollständig benetzt werden kann. Zudem kann während der Behandlung der Oberfläche die Salpetersäure beispielsweise durch Ausfällen des gelösten Eisens aufbereitet werden. Infolgedessen behält die Salpetersäure während der Behandlung der Oberfläche ihre Aktivität, so dass die Oberfläche nahezu rückstandsfrei von den Eisenpartikeln befreit werden kann.

In den folgenden Beispielen wurden Strahlpartikel STEELETTS GH der Firma WHEELABRATOR-ALLEVARD aus kantigen gebrochenen Stahlgussstrahlkömem mit einer chemischen Analyse C ≥ 0,85 %, P < 0,05 %, S < 0,05 % und einer Härte HV1 > 860 verwendet. Die verwendeten STEELETTS GH besaßen folgende Korngrößenverteilungen: GH-16 1,7-1,0 mm; GH-25 1,18-0,355 mm; GH-80 0,425-0,125 mm; GH-120 0,3-0,075 mm. Das Strahlen fand in einer Strahlkabine statt, in der der Strahldruck und der Abstand der Düse zu der zu behandelnden Oberfläche veränderbar war.

### Beispiel 1

Scheibenförmige Proben mit einem Durchmesser von 42 mm und einer Dicke von etwa 6 mm aus PROTASUL-100 (Ti-6Al-7Nb) und PROTASUL-Ti (Reintitan) wurden mit Strahlpartikeln GH-25 bei einem Strahldruck von 4,5 bar mit einem Abstand der Strahldüse zur Probe von 200 mm bestrahlt, bis die Oberfläche gleichmäßig mattgrau erschien. Anschließend wurden die Proben bei Raumtemperatur (21°C) jeweils in mechanisch gerührte 10 %-ige Lösungen der folgenden Säuren eingetaucht, d.h. gebeizt: Salpetersäure HNO₃, Phosphorsäure H₃PO₄, Schwefelsäure H₂SO₄.

Nach Beizzeiten von 30 und 60 Minuten wurden die Proben im Autoklav dampfsterilsiert (3 bar / 30 Minuten). Die Resteisenmenge auf den Proben in Form von Roststellen wurde in Abhängigkeit von der Säure und der Beizzeit halbquantitativ beurteilt. Die gleiche Behandlung wurde mit einer 10 %-igen Salzsäure HCl als Vergleichsbeispiel durchgeführt. Die Ergebnisse sind in der folgenden Tabelle zusammengefasst:

**Tabelle 1**

| Beizmittel | Beizzeit | Resteisen in Form von Rostflecken | | |
|---|---|---|---|---|
| | | keine | wenige | viele |
| H₂SO₄ | 30 | | | X |
| | 60 | | X | |
| H₃PO₄ | 30 | | | X |
| | 60 | | X | |
| HNO₃ | 30 | X | | |
| | 60 | X | | |
| HCl | 30 | | | X |
| | 60 | | | X |

Wie aus der Tabelle 1 ersichtlich ist, wird bei den Proben aus Reintitan und aus einer Titanlegierung das beste Resultat mit einer 10 %-igen Salpetersäure (HNO₃) erzielt, da bereits nach einer Beizzeit von 30 Minuten kein Resteisen in Form von Rostflecken nach der Autoklavbehandlung sichtbar war. Auch die Behandlung mit Phosphorsäure und mit Schwefelsäure ergab zufriedenstellende Ergebnisse, jedoch muss bei der Behandlung mit diesen Säuren eine Beizzeit von mindestens 60 Minuten, vorzugsweise 90 Minuten eingehalten werden, um eine Implantatoberfläche zu erhalten, die im wesentlichen frei von Resteisen ist.

### Beispiel 2

Proben aus PROTASUL-100 und PROTASUL-Ti mit den gleichen Spezifikationen wie die in Beispiel 1 benutzten Proben wurden mit Strahlpartikeln GH-80 bei einem Strahldruck von 4,5 bar und einem Abstand der Düse zur Probe von 200 mm gestrahlt. Als Beizmittel wurden Salpetersäure und Phosphorsäure mit einer Konzentration von 10 % und 20 % verwendet. Diese Lösungen wurden bei Raumtemperatur (21°C) mechanisch gerührt, die Proben wurden 30, 60 und 120 Minuten lang in die jeweiligen Lösungen eingetaucht. Anschließend wurden die Proben im Autoklaven dampfsterilisiert und wie in Beispiel 1 anhand von Rostflecken halbquantitativ auf Resteisen beurteilt. Zum Vergleich wurde außerdem die gleiche Behandlung mit einer 40 %-igen Salpetersäure und einer Beizzeit von 30 Minuten durchgeführt. Die Ergebnisse sind in der folgenden Tabelle zusammengefasst.

**Tabelle 2**

| Beizmittel | Beizzeit | Resteisen in Form von Rostflecken | | |
|---|---|---|---|---|
| | | keine | wenige | viele |
| H₃PO₄ | 30 | | X | |
| 10 % | 60 | | X | |
| | 120 | | X | |
| H₃PO₄ | 30 | | X | |
| 20 % | 120 | | X | |
| HNO₃ | 30 | X | | |
| 10% | 60 | X | | |
| | 120 | X | | |
| HNO₃ | 30 | X | | |
| 20 % | 120 | X | | |
| HNO₃, 40 % | 30 | | | X |

Nach der Behandlung mit der 10 %-igen Salpetersäure konnten bereits nach einer Beizzeit von 30 Minuten keine Rostflecken auf den gestrahlten Oberflächen beobachtet werden. Die Behandlung mit Phosphorsäure ergab ebenfalls zufriedenstellende Ergebnisse, da nur wenige Rostflecken beobachtet werden. Sowohl bei der Behandlung mit Salpetersäure als auch bei der Behandlung mit Phosphorsäure wirkt sich eine Konzentrationserhöhung des Lösungsmittels auf 20 % nicht negativ auf das Ergebnis aus.
Jedoch zeigt das Vergleichsbeispiel der Behandlung mit einer 40 %-igen HNO₃, dass sich die Steigerung der Salpetersäurekonzentration auf 40 % nachteilig auswirkt, denn nach der Dampfsterilisation waren noch viele Rostflecken erkennbar. Durch die mit der Konzentrationserhöhung verbundene Abnahme der Aktivität der Salpetersäure konnte das Resteisen nicht mehr zufriedenstellend weggebeizt werden.

### Beispiel 3

Scheibenförmige Proben aus PROTASUL-100 (Ti-6Al-7Nb), PROTASUL-64WF (Ti-6Al-4V) und PROTASUL-Ti (Reintitan) wurden mit den im Beispiel 2 verwendeten Strahlpartikeln GH-80 bei einem Strahldruck von 4,5 bar und einem Abstand der Düse zur Probe von 200 mm gestrahlt. Anschließend wurden die Oberflächen jeweils in 10 %-ige, mechanische gerührte HNO₃-Lösungen bei 21°C 15 und 30 Minuten lang eingetaucht. Danach wurden zur Sichtbarmachung der Eisenkontamination in Form von Rostflecken die Proben in einer Klimakammer einem Kondenswasser-Prüfklima von 40°C und 100 % Luftfeuchte eine Stunde lang ausgesetzt. Als Vergleichsbeispiel wurden zu den mit HNO₃ gebeizten Proben auch ungebeizte Proben in den Klimaschrank gegeben.

Nach der Behandlung in der Klimakammer waren die ungebeizten Proben mit unzähligen Rostflecken überdeckt. Bei den Proben, die 15 Minuten lang mit HNO₃ behandelt wurden, waren vereinzelte Rostflecken zu beobachten, und nach einer Behandlungszeit von 30 Minuten war keine Eisen-Kontamination mehr vorhanden.

### Beispiel 4

Je vier Proben aus PROTASUL-Ti, PROTASUL-64WF und PROTASUL-100 mit Durchmesser 42 mm wurden mit STEELETTS GH-16, GH-25, GH-80 und GH-120 bei 4,5 bar Strahldruck und einem Abstand der Düse von den jeweiligen Proben von ca. 200 mm gestrahlt. Anschließend wurde mit 10 %-iger Salpetersäure, die bei Raumtemperatur (21°C) mechanisch gerührt wurde, unter Einhaltung folgender Beizzeit behandelt: 30 Minuten für STEELETTS GH-80. Anschließend wurden jeweils zwei Proben in 250 ml 10 %-iger HNO₃ über 5 Stunden gelagert. Als Vergleichsproben dienten zwei ungestrahlte Proben. Danach wurde der Eisengehalt in den jeweiligen HNO₃-Lösungen bestimmt.

Für die ungestrahlten Referenzproben ergab sich ein Eisengehalt der jeweiligen HNO₃-Lösungen von kleiner als 0,2 mg/l. Der Eisengehalt der HNO₃-Lösungen, in denen die gestrahlten Proben gelagert wurden, betrug 1,4 mg/l. Die Eisen-Kontamination beträgt somit 0,0126 mg Fe/cm². Demnach sind die Restkontaminationen der gestrahlten Proben so gering, dass sie vernachlässigt werden können. Ein zusätzlicher Sichttest auf Eisenkontamination nach einer einstündigen Behandlung in der Klimakammer bei einem Kondenswasser-Prüfklima von 40°C und 100 % Luftfeuchtigkeit, der vor dem Lagern in HNO₃ auf der Oberfläche durchgeführt wurde, ergab für die gestrahlten Proben, dass keine Rostflecken erkennbar waren.

### Beispiel 5

Scheibenförmige Proben aus PROTASUL-Ti, PROTASUL-64WF und PROTASUL-100 wurden mit STEELETTS GH-16, GH-25, GH-80 und GH-120 gestrahlt. Anschließend wurden die mit den unterschiedlichen STEELETTS GH bestrahlten Proben wie unter Beispiel 3 beschrieben mit 10 %-iger Salpetersäure behandelt. Die Oberflächenrauhigkeit wurde mit einem mechanischen Abtastgerät der Firma Perthen, Perthometer S5P, mit einem Abtaster RHTR2-50 bei einer Messlänge von 4,8 mm und einer Grenzwellenlänge von 0,8 mm gemessen. Die Ergebnisse sind in den folgenden Tabellen zusammengefasst:

**Tabelle 3**

| STEELETTS GH-120 | | | | | | | |
|---|---|---|---|---|---|---|---|
| Werkstoff | Strahldruck (bar) | Oberflächenrauhigkeit (µm) | | | | | |
| | | vor dem Beizen | | | nach dem Beizen | | |
| | | Ra | Rz | Rmax | Ra | Rz | Rmax |
| P-Ti | 1,0 | 3,0 | 20 | 24 | 2,6 | 19 | 23 |
| P-100 | 1,0 | 2,9 | 19 | 21 | 2,9 | 18 | 23 |
| P-Ti | 4,5 | 5,2 | 30 | 35 | 5,0 | 29 | 32 |
| P-100 | 4,5 | 5,3 | 31 | 35 | 4,8 | 28 | 32 |

**Tabelle 4**

| STEELETTS GH-80 | | | | | | | |
|---|---|---|---|---|---|---|---|
| Werkstoff | Strahldruck (bar) | Oberflächenrauhigkeit (µm) | | | | | |
| | | vor dem Beizen | | | nach dem Beizen | | |
| | | Ra | Rz | Rmax | Ra | Rz | Rmax |
| P-Ti | 4,5 | 7,1 | 41 | 60 | 5,2 | 30 | 33 |
| P-64WF | 4,5 | 6,6 | 36 | 42 | 5,1 | 30 | 35 |
| P-100 | 4,5 | 5,1 | 37 | 50 | 5,0 | 29 | 32 |

**Tabelle 5**

| STEELETTS GH-25 | | | | | | | |
|---|---|---|---|---|---|---|---|
| Werkstoff | Strahldruck (bar) | Oberflächenrauhigkeit (µm) | | | | | |
| | | vor dem Beizen | | | nach dem Beizen | | |
| | | Ra | Rz | Rmax | Ra | Rz | Rmax |
| P-Ti | 4,5 | 11 | 66 | 80 | 7,6 | 41 | 54 |
| P-64WF | 4,5 | 11 | 53 | 60 | 6,5 | 42 | 51 |
| P-100 | 4,5 | 9 | 51 | 83 | 7,7 | 43 | 53 |

**Tabelle 6**

| STEELETTS GH-16 | | | | | | | |
|---|---|---|---|---|---|---|---|
| Werkstoff | Strahldruck (bar) | Oberflächenrauhigkeit (µm) | | | | | |
| | | vor dem Beizen | | | nach dem Beizen | | |
| | | Ra | Rz | Rmax | Ra | Rz | Rmax |
| P-Ti | 4,5 | 13 | 66 | 85 | 14 | 70 | 90 |
| P-64WF | 4,5 | 13 | 65 | 95 | 12 | 59 | 76 |
| P-100 | 4,5 | 14 | 72 | 96 | 12 | 58 | 73 |

Wie aus den Tabellen ersichtlich ist, sind die Rauhigkeitswerte vor dem Beizen ein wenig höher als nach dem Beizen. Dies resultiert daraus, dass durch die Behandlung mit Salpetersäure die Eisenkontaminationen abgetragen werden, der Titanwerkstoff jedoch praktisch nicht angegriffen wird, d.h. nur in einem Ausmaß, das auf die für den praktischen Einsatz erforderliche Rauhigkeit keinen negativen Einfluss hat. Die Proben, die mit GH-25, GH-80 und GH-120 gestrahlt wurden, ergaben zufriedenstellende Rauhigkeitswerte. Die mit STEELTEETS GH-16 gestrahlten Prüfplättchen zeigten auch nach 3 Stunden Beizzeit einzelne Rostflecken nach einer Behandlung im Autoklaven wie in Beispiel 1 beschrieben. Daher muss für Oberflächen, die mit GH-16 bestrahlt sind und daher sehr hohe Rauhigkeitswerte aufweisen, die Beizzeit auf ca. 4 Stunden verlängert werden.

### Beispiel 6

An stangenförmigen Proben aus PROTASUL-100 mit 4 mm Durchmesser wurde die Umlaufbiegewechselfestigkeit bestimmt. Zuvor wurde die Oberfläche der Proben mit STEELETTS GH-80 gestrahlt und wie unter Beispiel 3 beschrieben behandelt. Es ergab sich eine Oberflächenrauhigkeit der Proben Ra 5 bis 6 µm und Rz 30 bis 35 µm.

Zum Vergleich wurde eine entsprechende PROTASUL-100 Probe mit Korund gestrahlt und dem gleichen Test unterworfen. In der folgenden Tabelle sind die Testergebnisse aufgeführt:

**Tabelle 7**

| Umlaufbiegewechselfestigkeit (10⁷ Zyklen) | (MPa) |
|---|---|
| STEELETTS gestrahlt / PROTASUL-100 | ca. 460 |
| Korund gestrahlt / PROTASUL-100 | ca. 500 |

Die Umlaufbiegewechselfestigkeit von erfindungsgemäß gestrahlten und behandelten Proben sind im Vergleich zu den lediglich mit Korund gestrahlten Proben rund 10 % geringer, jedoch immer noch zufriedenstellend im Hinblick auf die in der Praxis für Implantate erforderliche Umlaufbiegewechselfestigkeit.

### Beispiel 7

Es wurden dynamische Festigkeitsuntersuchungen von mit STEELTETTS gestrahlten Oberflächen an Hüftprothesenmodellen von ZWEYMÜLLER, SPOTORNO und MÜLLER durchgeführt. Ausgangsmaterial waren mechanisch fertig bearbeitete Prothesen, die wie unter Beispiel 1 beschrieben gestrahlt, gebeizt (d.h. 30 Minuten lang mit 10 %-iger HNO₃ behandelt) und auf Eisenkontaminationen geprüft wurden. Anschließend wurden die Proben gewaschen und die Oberflächenrauhigkeit gemessen. Zum Vergleich wurde eine ZWEYMÜLLER-Hüftprothese mit Korund gestrahlt.

ZWEYMÜLLER: Hüftprothese, Gr. 1 (Art. Nr. 2841):
Nach dem Beizen wurden bei der Prothese Rostflecken in den proximalen Löchern festgestellt. Nach Substitution von ca. 30 % Wasser durch Industriealkohol senkte sich die Oberflächenspannung der Beizlösung, so dass nachfolgend gebeizte Schäfte von ZWEYMÜLLER-Prothesen auch in den proximalen Löchern rostfrei waren.

SPOTORNO-Hüftprothese, Gr. 7 (Art. Nr. 29.00.09-070):
Als Beizlösung wurde eine 10 %-ige Salpetersäure ohne Alkohol verwendet. Nach dem Beizen konnten Rostflecken im medialen Ausschlagloch erkannt werden.

MÜLLER-Geradschaft, Gr. 7,5 (Artl. Nr. 23.00.59-075):
Auch hier wurde ohne Alkohol gebeizt und es wurden Rostflecken nach dem Beizen im medialen Ausschlagloch festgestellt.

**Tabelle 8**

| | Oberflächenrauhigkeit | | |
|---|---|---|---|
| **ZWEYMÜLLER:** | | | |
| STEELETTS GH-80 gestrahlt | Ra 5,2 µm | Rz 31 µm | Rmax 39 µm |
| Korund gestrahlt | Ra 5,4 µm | Rz 32 µm | Rmax 43 µm |

| **SPOTORNO:** | | | |
|---|---|---|---|
| STEELETTS GH-80 gestrahlt | Ra 4,7 µm | Rz 29 µm | Rmax 37 µm |

| **MÜLLER**: | | | |
|---|---|---|---|
| STEELETTS GH-80 gestrahlt | Ra 5,2 µm | 30 µm | Rmax 36 µm |

Die dynamische Festigkeitsuntersuchungen wurden nach der Norm ISO 7206/3 (ohne seitliche Schaftneigung) und nach der Norm ISO/DIS 7206/4 (mit 9° seitlicher Neigung) durchgeführt (Versuchsende: 5 Millionen Zyklen).

**Tabelle 9**

| **ZWEYMÜLLER** | ISO 7206/3 (0°) | ISO/DIS 7206/4 (9°) |
|---|---|---|
| STEELETTS GH-80 | 300/5800N | 300/3800N |
| Korund gestrahlt | 300/5800N | 300/3800N |

**Tabelle 10**

| **SPORTORNO** | ISO 7206/3 (0°) | ISO/DIS 7206/4 (9°) |
|---|---|---|
| STEELETTS GH-80 | nicht geprüft | 300/4800N |
| Korund gestrahlt | 300/6300N | 300/5300N |

**Tabelle 11**

| **MÜLLER** | ISO 7206/3 (0°) | ISO/DIS 7206/4 (9°) |
|---|---|---|
| STEELETTS GH-80 | 300/3800N | 300/8300N |
| Korund gestrahlt | 300/3800N | 300/8300N |

Wie aus den Tabellen ersichtlich ist, ist die dynamische Festigkeit der geprüften ZWEYMÜLLER-Prothesen im simuliert gelockerten Zustand für Oberflächen, die mit STEELETTS und mit Korund gestrahlt wurden, gleich hoch.

Die mit STEELETTS gestrahlte SPORTORNO-Hüftschaft-Prothese wurde nur nach der Norm ISO/DIS 7206/4 geprüft. Als dauernd ertragbare Last wurde 300/4800N ermittelt.

Die MÜLLER-Geradschaftprothese wurde im simuliert gelockerten Zustand nach der Norm ISO/DIS 7206/4 geprüft. Bei der Last von 300/3800N trat über 5 Millionen Zyklen kein Bruch auf. Zusätzlich wurden Halstests mit voll einzementierter Prothese nach ISO/DIS 7206/4 durchgeführt. Die Müller-Schäfte durchliefen die Schwellbelastung von 300/8300 Newton mehr als 10 Millionen Zyklen lang ohne Bruch.

Insgesamt konnten bei den untersuchten Hüftprothesen von ZWEYMÜLLER, SPOTORNO und MÜLLER keine signifikanten Festigkeitsunterschiede zwischen Oberflächen, die mit STEELETTS gestrahlt wurden, und Oberflächen, die mit Korund gestrahlt wurden, festgestellt werden.

### Beispiel 8

Es wurden potentio-dynamische Korrosionsversuche an einem Strahlmittel und an Reintitan gemäß dem Standard ASTM G5 durchgeführt, wobei folgende Parameter gewählt wurden: Abtast-Rate 10 mV/min, 0,9%-ige NaCl-Lösung mit einem pH von 4, Temperatur der NaCl-Lösung 40°C. Der pH des NaCl-Elektrolyten wurde mit 10%-iger HCl-Lösung eingestellt. Während des gesamten Versuches wurde die NaCl-Lösung mit Stickstoff durchperlt. Als Gegenelektrode wurde eine Satured Calomel Electrode (SCE) verwendet.

Der Prüfling wurde an ein Elektrokabel angeschlossen und in einen selbsthärtenden Kunststoff eingebettet. Die Oberflächenpräparation erfolgte mittels Schleifen bis Körnung 1200.

### Strahlmittel GH-80

Stahlgussstrahlkörner STEELETTS GH-80 wurden zu einem Prüfling verarbeitet und im Rahmen des potentio-dynamischen Korrosionsversuchs untersucht. Dieses Strahlmittel ist ein übereutektoider Stahl mit einem Kohlenstoffanteil von > 0,85 %. Um eine elektrochemische Charakterisierung vornehmen zu können, wurden Schmelzproben hergestellt. Dazu wurde das pulvrige Strahlmittel in einem Schmelzprozess im Vakuum (Vacumet AG, Winterthur) zu einer Probe mit einem Durchmesser von etwa 20 mm und einer Höhe von etwa 10 mm verarbeitet.

Der potentio-dynamische Korrosionsversuch ergab, dass das Ruhepotential des Strahlmittels GH-80 bei etwa -850 mV liegt. Mit zunehmendem Potential steigt die Stromdichte, wobei die Auflösungsrate im Potentialbereich von -800 mV bis 0 mV stark ansteigt, jedoch mit weiter zunehmendem Potential weniger stark zunimmt. Bei einem Potential von +800 mV liegt die Stromdichte bei etwa 0,1 A/cm² bzw. 100.000 µA/cm². Nach dem Versuchsende war die gesamte Prüflösung wegen der massiven Auflösung des Eisens schwarz-braun verfärbt.

### Reintitan

Als Referenzprobe wurde Reintitan, Reinheitsgrad 4, ebenfalls im potentio-dynamischen Versuch unter gleichen Bedingungen geprüft.

Es wurde festgestellt, dass das Ruhepotential bei etwa -550 mV liegt. Ab etwa -300 mV beginnt der Passivbereich, d.h. der Bereich, in dem die Stromdichte unabhängig vom zunehmendem Potential konstant bleibt. Die Stromdichte in diesem Bereich liegt bei etwa 1 µA/cm².

Anschließend wurden potentio-statische Korrosionsversuche an mit Strahlmittel kontaminierten Reintitanproben durchgeführt. Dazu wurden wie beim potentio-dynamischen Korrosionsversuch die Reintitanproben eingebettet und geschliffen. Mit einer gehärteten Metallspitze wurden einzelne, kleine Löcher ins Reintitan gehämmert. Die Löcher wurden dann mit einzelnen Strahlmittelkörnern GH-80 gefüllt und anschließend mit der gleichen Metallspitze, welche für die Löcher verwendet wurde, in die Vertiefung getrieben.

Bei den potentio-statischen Versuchen wurde die Probe einem konstanten Potential ausgesetzt. Der Versuch wurde bei einem Potential von +300 mV durchgeführt und die resultierende Stromdichte wurde zwei Stunden lang gemessen. Die restlichen Versuchsparameter sind identisch mit denjenigen der potentio-dynamischen Korrosionsversuche.

Die potentio-statischen Versuche ergaben, dass die Stromdichte mit der Zeit abnahm. Nach zwei Stunden konnten auf der Titanoberfläche nur noch vereinzelte Eiseneinschlüsse nachgewiesen werden. Infolge dessen zeigen die Versuche, dass eine Reinigungswirkung erzielt wurde.

Aus den Versuchen des Beispiels 8 folgt, dass eine mit Stahlgussstrahlkörnern kontaminierte Reintitan-Oberfläche durch eine elektrolytische Behandlung selektiv von den Strahlkörnern befreit werden kann. Die zuerst durchgeführte elektrochemische Charakterisierung im Rahmen der potentio-dynamischen Korrosionsversuche belegt, dass sich die Normalpotentiale der Materialien der Strahlpartikel und der Titanoberfläche ausreichend unterscheiden, um nur die Eisen-Strahlpartikel elektrolytisch angreifen zu können. Die potentio-statischen Versuche ergaben ferner, dass bei der elektrolytischen Behandlung eine Reinigungswirkung dadurch erzielt wird, dass Eisenkontaminationen in einer Reintitanoberfläche selektiv aufgelöst bzw. abgelöst wurden.

Die elektrolytische Behandlung, beispielsweise das oben beschriebene potentio-statische Verfahren, kann auch kombiniert mit einer chemischen Behandlung der Oberfläche durchgeführt werden. Auf diese Weise kann die Reinigungswirkung verbessert werden. Als kombiniertes chemisches und elektrolytisches Bad können Elektrolyten auf wässriger Basis, beispielsweise säurehaltige Elektrolyten, verwendet werden. Das Potential kann zur Reinigung einer Reintitanoberfläche von Eisen-Strahlpartikeln im Bereich von -500 mV bis +800 mV eingestellt werden. Um zu vermeiden, dass ein zu niedriges Potential den Stahl nur langsam auflöst und ein zu hohes Potential die Titan-Oberfläche beschädigt, ist jedoch ein Potential im Bereich von +100 mV bis +500 mV, insbesondere ein Potential von +300 mV vorzuziehen.
Die Beispiele zeigen, dass durch das erfindungsgemäße Verfahren ein medizinisches Implantat hergestellt werden kann, insbesondere eine Gelenkprothese, z.B. eine Hüftgelenkprothese, die eine kontaminationsfreie Titanoberfläche aufweist. Die Rauhigkeit der mit STEELETTS GH-80 gestrahlten Oberflächen ist vergleichbar mit herkömmlichen, mit Korund gestrahlten Oberflächen. Sogar höhere Rauhigkeitswerte sind mit entsprechend gröberem Stahlschrot erzielbar. Signifikante Festigkeitsunterschiede, gemessen an Umlaufbiegeproben und Hüftprothesen gleicher Rauhigkeit, konnten zwischen Oberflächen, die mit STEELETTS gestrahlt wurden, und Oberflächen, die mit Korund gestrahlt wurden, nicht festgestellt werden.

Zusammenfassend lässt sich feststellen, dass die durch das erfindungsgemäße Verfahren hergestellten Implantatoberflächen und Implantate alle Anforderungen erfüllen, die für Prothesen im medizinischen Bereich erforderlich sind. Zusätzlich sind die erfindungsgemäß hergestellten, gestrahlten Oberflächen nahezu kontaminationsfrei, so dass Komplikationen durch "vagabundierende", d.h. sich nach dem Implantieren ablösende Strahlpartikel vermieden werden können.

## Patentansprüche

1. Verfahren zur Herstellung eines Implantats, insbesondere eines metallischen Implantats, mit den Schritten
Aufrauhen der Oberfläche des Implantats durch Strahlen mit Strahlpartikeln und
Behandeln der Oberfläche mit einem Lösungsmittel, das selektiv die Strahlpartikel löst,
**dadurch gekennzeichnet,**
**dass** das Material der Oberfläche Titan oder eine Titan-Legierung aufweist,
**dass** die Strahlpartikel Eisenpartikel umfassen, und
**dass** das Lösungsmittel HNO₃ aufweist.

2. Verfahren zum Dekontaminieren einer mit Strahlpartikeln behandelten Oberfläche, insbesondere einer mit Strahlpartikeln behandelten metallischen Implantatoberfläche, bei dem
die Oberfläche mit einem Lösungsmittel behandelt wird, das selektiv die Strahlpartikel löst,
**dadurch gekennzeichnet,**
**dass** das Material der Oberfläche Titan oder eine Titan-Legierung aufweist,
**dass** die Strahlpartikel Eisenpartikel umfassen, und
**dass** das Lösungsmittel HNO₃ aufweist.

3. Verfahren nach Anspruch 1 oder 2, bei dem das Behandeln der Oberfläche ein Eintauchen der Oberfläche in das Lösungsmittel umfasst.

4. Verfahren nach einem der vorstehenden Ansprüche, bei dem vor dem Behandeln mit dem Lösungsmittel eine Reinigung der Oberfläche erfolgt.

5. Verfahren nach einem der vorstehenden Ansprüche, bei dem das Lösungsmittel beim Behandeln der Oberfläche aufbereitet wird.

6. Verfahren nach einem der vorstehenden Ansprüche, bei dem die Strahlpartikel gehärtete Eisenpartikel umfassen.

7. Verfahren nach einem der vorstehenden Ansprüche, bei dem das Behandeln der Oberfläche ein etwa 30-minütiges Eintauchen der Oberfläche in eine mechanisch gerührte, etwa 10%-ige HNO₃ als Lösungsmittel bei Raumtemperatur umfasst.

8. Verfahren nach Anspruch 7, bei dem
vor dem Behandeln der Oberfläche eine Reinigung der Oberfläche mit Industriealkohol erfolgt.

9. Verfahren nach einem der vorstehenden Ansprüche, bei dem das Lösungsmittel während des Behandelns der Oberfläche durch eine Fällung der gelösten Strahlpartikel, insbesondere durch eine Eisenfällung, aufbereitet wird.

## Claims

1. A method for the manufacture of an implant, in particular of a metallic implant comprising the steps of
roughening the surface of the implant by blasting with blasting particles; and
treating the surface with a solvent which selectively dissolves the blasting particles,
**characterised in that** the surface comprises titanium or a titanium alloy;
**in that** the blasting particles include iron particles; and
**in that** the solvent comprises HNO₃.

2. A method for the decontamination of a surface treated with blasting particles, in particular of a metallic implant surface treated with blasting particles, wherein the surface is treated with a solvent which selectively dissolves the blasting particles,
**characterised in that** the surface comprises titanium or a titanium alloy;
**in that** the blasting particles include iron particles; and
**in that** the solvent comprises HNO₃

3. A method in accordance with claim 1 or claim 2, in which the treatment of the surface includes an immersion of the surface into the solvent

4. A method in accordance with any one of the preceding claims, in which a cleaning of the surface takes place before the treatment with the solvent.

5. A method in accordance with any one of the preceding claims, in which the solvent is prepared during the treatment of the surface.

6. A method in accordance with any one of the preceding claims, in which the blasting particles include hardened iron particles.

7. A method in accordance with any one of the preceding claims, in which the treatment of the surface includes an approximately 30 minute immersion of the surface into a mechanically stirred, approximately 10% HNO₃ solution at room temperature.

8. A method in accordance with claim 7, in which a cleaning of the surface with industrial alcohol takes place before the treatment of the surface.

9. A method in accordance with any one of the preceding claims, in which the solvent is prepared during the treatment of the surface by a precipitation of the dissolved blasting particles, in particular by an iron precipitation.

## Revendications

1. Procédé de fabrication d'un implant, en particulier d'un implant métallique, comprenant les étapes qui consistent
à rendre rugueuse la surface de l'implant par projection d'un jet de particules, et
à traiter la surface avec un solvant qui dissout sélectivement les particules projetées,
**caractérisé**
**en ce que** le matériau de la surface comporte du titane ou un alliage de titane,
**en ce que** les particules projetées comprennent des particules de fer, et
**en ce que** le solvant comporte du HNO₃.

2. Procédé de décontamination d'une surface traitée avec un jet de particules, en particulier d'une surface d'implant métallique traitée avec un jet de particules, dans lequel :
la surface est traitée avec un solvant qui dissout sélectivement les particules projetées,
**caractérisé**
**en ce que** le matériau de la surface comporte du titane ou un alliage de titane,
**en ce que** les particules projetées comprennent des particules de fer, et
**en ce que** le solvant comporte du HNO₃.

3. Procédé suivant la revendication 1 ou 2, dans lequel le traitement de la surface comprend une immersion de la surface dans le solvant.

4. Procédé suivant l'une des revendications précédentes, dans lequel un nettoyage de la surface est effectué avant le traitement avec le solvant.

5. Procédé suivant l'une des revendications précédentes, dans lequel le solvant est préparé au moment du traitement de la surface.

6. Procédé suivant l'une des revendications précédentes, dans lequel les particules projetées comprennent des particules de fer trempé.

7. Procédé suivant l'une des revendications précédentes, dans lequel le traitement de la surface comprend une immersion de la surface pendant environ 30 minutes à la température ambiante dans du HNO₃ à environ 10 %, agité mécaniquement, utilisé comme solvant.

8. Procédé suivant la revendication 7, dans lequel un nettoyage de la surface avant son traitement est effectué avec de l'alcool industriel.

9. Procédé suivant l'une des revendications précédentes, dans lequel le solvant est préparé pendant le traitement de la surface par une précipitation des particules projetées dissoutes, en particulier par une précipitation du fer.
